# EUROPEAN PATENT APPLICATION

(11) **EP 1 219 302 A1**
(43) Date of publication of application: **03.07.2002**
(21) Application number: 00925627.2
(22) Date of filing: 11.05.2000
(51) Int. Cl.: A61K 45/00, A61K 31/155, A61K 31/095, A61K 31/44, A61K 31/74, A61K 33/00, A61K 33/08, A61K 33/44, A61K 38/06, A61K 31/198, A61M 1/16, A61M 1/36, A61P 7/08

(54) **TRAPPING AGENT FOR BLOOD CARBONYL COMPOUNDS**

(30) Priority: 12.05.1999 JP 13197899
(71) Applicant: Kurokawa, Kiyoshi, Sinjuku-ku, Tokyo 162-0061 (JP); Miyata, Toshio, Isehara-shi, Kanagawa 259-1132 (JP)
(72) Inventor: MIYATA, Toshio, Isehara-shi, Kanagawa 259-1132 (JP)
(74) Representative: Grünecker, Kinkeldey, Stockmair & Schwanhäusser Anwaltssozietät
(86) International application number: JP0003029
(87) International publication number: WO0069466

(57) **Abstract**

A carbonyl compound-trapping agent is contacted with a patient's blood. Thereby, carbonyl compounds are effectively removed from the patient's blood, and thus, damages from carbonyl compounds (i.e. carbonyl stress) can be reduced.

## Description

### Technical Field

The present invention relates to the removal of blood carbonyl compounds, specifically, to the removal of blood carbonyl compounds using a carbonyl compound-trapping agent.

### Background Art

Hemodialysis is a typical therapy used for treating patients with chronic renal failure, wherein blood waste products and toxic substances are removed by contacting blood with a dialysate via a semipermeable membrane. However, the disease state of renal failure cannot completely be inhibited by dialysis. Such a disease state includes increase of advanced glycation end products (AGEs) and carbonyl intermediate (precursors of AGE) levels in renal failure patients. AGEs have been reported to modify protein structure and function and to be involved in the onset of complications of dialysis, such as dialysis amyloidosis and arteriosclerosis (Makita, Z. et al., N. Engl. J. Med., 325: 836-842, 1991; Miyata, T. et al., J. Clin. Invest., 92: 1243-1252, 1993; Miyata, T. et al., J. Clin. Invest., 93: 521-528, 1994; Miyata, T. et al., Proc. Natl. Acad. Sci. USA, 93: 2353-2358, 1996; Horie, K. et al., J. Clin. Invest., 100: 2995-3004, 1997; Miyata, T. et al., FEBS letters, 445: 202-206, 1999). Recently, it was revealed that the accumulation of carbonyl intermediates such as glyoxal, methylglyoxal, 3-deoxy glucosone and arabinose (so-called, carbonyl stress) (Odani et al., Biochem. Biophys. Res. Commun., 256: 89-93, 1999; Niwa et al., Nephron, 69: 438-443, 1995; Miyata et al., Kidney Int., 55: 389-399, 1999; Miyata et al., J. Am. Soc. Nephrol., 9: 2349-2356, 1998) in blood plasma results in an increased AGE level in renal failure (Miyata, T. et al., J. Am. Soc. Nephrol., 9: 2349-2356, 1998; Miyata, T. et al., Kidney Int., 55: 389-399, 1999). A variety of carbonyl intermediates (AGE precursors) derive mainly from carbohydrates and lipids (Miyata, T. et al., Kidney Int., 55: 389-399, 1999; Miyata, T. et al., Kidney Int., 54; 1290-1295, 1998; Miyata, T. et al., Kidney Int., 51: 1170-1181, 1997). Conventional hemodialysis cannot effectively remedy these increased levels of AGE and carbonyl intermediates, namely "carbonyl stress", in renal failure patients.

### Disclosure of the Invention

An objective of the present invention is to provide a carbonyl compound-trapping agent for removing blood carbonyl compounds. Another objective of the present invention is to provide a method and agent for improving carbonyl stress state in a living body. The present invention enables to prevent the damage caused by carbonyl compounds in hemodialysis patients who are particularly prone to fall into a carbonyl stress state. The objective of the present invention is to reduce the damage by carbonyl compounds as much as possible in hemodialysis patients.

First, the present inventors studied how the hemodialysis membrane used for hemodialysis influenced the quantity of carbonyl compounds in patients'. blood. The content of blood pentosidine, a marker for the accumulation of carbonyl intermediate products (i.e. carbonyl stress), was compared by quantification with high-performance liquid chromatography (HPLC) for each type of dialysis membrane used by dialysis patients. The result showed that free pentosidine was markedly removed by dialysis through any dialysis membrane, while protein-bound pentosidine, which occupies the major portion of pentosidine in the body could not be effectively removed by dialysis.

Comparing the type of dialysis membrane indicated no difference in the value of protein-bound and free pentosidine with low-flux cellulosic, high-flux polymethyl methacrylate (PMMA) and AN69, but the value was lower with high-flux polysulfone (PS) (p<0.01). There were no differences of the value depending on whether the patient was Japanese or Belgian, or on the manufacturer of the PS membrane. Switching the dialysis membrane used by three patients from AN69 to PS decreased the protein-bound pentosidine level, and then, return to AN69 increased the level back to the original. These results revealed that polysulfone membrane is effective as a dialysis membrane for suppressing the generation of carbonyl compounds.

Then, the present inventors conceived the utilization of a carbonyl compound-trapping agent for the effective removal of blood carbonyl compounds. Blood plasma prepared from the blood of dialysis patients was incubated with carriers on which a carbonyl compound-trapping agent had been immobilized, and blood carbonyl compounds were quantified. The result showed that the incubation with carriers on which a carbonyl compound-trapping agent had been immobilized significantly reduced the quantities of blood carbonyl compounds.

Based on these findings, the present inventors focused on carbonyl compounds accumulated in blood and thought that the removal of carbonyl compounds accumulated in blood was required for improving carbonyl stress, mainly protein modification, in dialysis patients. Then, the inventors found that the use of a compound having the function of eliminating or reducing the protein modification activity of carbonyl compounds by chemically reacting with or adsorbing carbonyl compounds was effective to achieve the objective, and thus completed the present invention. In the present invention, a carrier, on which a compound having such a function has been immobilized, or the compound itself, is called a "carbonyl compound-trapping agent".

Namely, the present invention relates to a carbonyl compound-trapping agent for removing blood carbonyl compounds, as well as a method and agent for improving carbonyl stress state in a living body as described below:
(1) a carbonyl compound-trapping agent that removes a blood carbonyl compound;
(2) the carbonyl compound-trapping agent according to (1), wherein said agent is used in hemodialysis;
(3) the carbonyl compound-trapping agent according to (1), wherein said agent is immobilized on a blood-insoluble carrier;
(4) the carbonyl compound-trapping agent according to (3), wherein said carrier is a dialysis membrane;
(5) the carbonyl compound-trapping agent according to (4), wherein said dialysis membrane is a polysulfone membrane;
(6) the carbonyl compound-trapping agent according to (1), wherein said carbonyl compound-trapping agent is a Maillard reaction inhibitor;
(7) the carbonyl compound-trapping agent according to (6), wherein said Maillard reaction inhibitor comprises at least one compound selected from the group consisting of aminoguanidine, pyridoxamine, hydrazine, SH group-containing compound, and derivatives thereof;
(8) the carbonyl compound-trapping agent according to (1), wherein said agent comprises a compound that is insoluble in blood;
(9) the carbonyl compound-trapping agent according to (8), wherein said compound that is insoluble in blood comprises at least one compound selected from the group consisting of an ion exchange resin, activated carbon, silica gel, alumina, and calcium carbonate;
(10) an agent for improving the carbonyl stress state in a living body, wherein said agent comprises a carbonyl compound-trapping agent as an active ingredient;
(11) an agent for improving the carbonyl stress state in blood, wherein said agent comprises a carbonyl compound-trapping agent as an active ingredient;
(12) the agent for improving the carbonyl stress state according to (11), wherein said agent is immobilized within the blood circuit;
(13) the agent for improving the carbonyl stress state according to (11), wherein the carbonyl compound-trapping agent is a Maillard reaction inhibitor;
(14) the agent for improving the carbonyl stress state according to (13), wherein said Maillard reaction inhibitor comprises at least one compound selected from the group consisting of aminoguanidine, pyridoxamine, hydrazine, SH group-containing compound, and derivatives thereof;
(15) a method for improving carbonyl stress state, wherein said method comprises the step of contacting, within the blood circuit, a patient's blood with a carbonyl compound-trapping agent;
(16) the method according to (15), wherein said method comprises the step of immobilizing said carbonyl compound-trapping agent on a blood-insoluble carrier.

The present invention also relates to the use of a carbonyl compound-trapping agent for removing blood carbonyl compounds. Further, the present invention relates to the use of a carbonyl compound-trapping agent for manufacturing an agent for improving blood carbonyl stress.

In the present invention, carbonyl compounds to be trapped include, for example, the following compounds that accumulate in the blood of renal failure patients together with oxidative stress. Carbonyl compounds derived from carbohydrates:
· arabinose
· glyoxal
· methylglyoxal
· 3-deoxyglucosone

Carbonyl compound derived from ascorbic acid:
· dehydroascorbic acid

Carbonyl compound derived from lipid:
· hydroxynonenal
· malondialdehyde
· acrolein

A preferred carbonyl compound-trapping agent in the present invention is one capable of completely inhibiting or reducing the protein-modification activity of all these carbonyl compounds through a chemical reaction or adsorption. However, the carbonyl compound-trapping agent of the present invention also includes an agent effective for the major carbonyl compounds among these. Carbonyl compound-trapping agents that can be used in the present invention include, for example, the following compounds:
· aminoguanidine (Foote, E. F. et al., Am. J. Kidney Dis., 25: 420-42 (1995))
· · ± 2-isopropylidenehydrazono-4-oxo-thiazolidin-5-ylacetanilide (OPB-9195; S. Nakamura, 1997, Diabetes 46:895-899)

Further, the carbonyl compound-trapping agent includes, for example, the following compounds or derivatives thereof that are capable of functioning as carbonyl compound-trapping agents. "Derivatives" indicate compounds having an atomic or molecular substitution (s) at any position as compared with the parent compound. By linking to carriers to facilitate separation from blood, these compounds can be used as carbonyl compound-trapping agents in the present invention. Alternatively, if the compound itself is insoluble in blood, it can be used as the carbonyl compound-trapping agent of this invention without being immobilized on carriers.
(1) guanidine derivatives such as methylguanidine (JP-A Sho 62-142114; JP-A Sho 62-249908; JP-A Hei 1-56614; JP-A Hei 1-83059; JP-A Hei 2-156; JP-A Hei 2-765; JP-A Hei 2-42053; JP-A Hei 6-9380; Published Japanese Translation of International Publication 5-505189), etc.
(2) hydrazine derivatives such as sulfonylhydrazine, etc.
(3) five-membered heterocyclic compounds having two nitrogen atoms, such as pyrazolone (JP-A Hei 6-287179), pyrazoline (JP-A Hei10-167965), pyrazole (JP-A Hei 6-192089; JP-A Hei6-298737; JP-A Hei 6-298738), imidazolidine (JP-A Hei 5-201993; JP-A Hei 6-135968; JP-A Hei7-133264; JP-A Hei 10-182460), hydantoin (JP-A Hei 6-135968), etc.
(4) five-membered heterocyclic compounds having three nitrogen atoms, such as triazole (JP-A Hei 6-192089), etc.
(5) five-membered heterocyclic compounds having a nitrogen atom and a sulfur atom, such as thiazoline (JP-A Hei 10-167965), thiazole (JP-A Hei 4-9375; JP-A Hei 9-59258), thiazolidine (JP-A Hei 5-201993; JP-A Hei 3-261772; JP-A Hei 7-133264; JP-A Hei 8-157473), etc.
(6) five-membered heterocyclic compounds having a nitrogen atom and an oxygen atom, such as oxazole (JP-A Hei 9-59258), etc.
(7) nitrogen-containing six-membered heterocyclic compounds such as pyridine (JP-A Hei 10-158244; JP-A Hei 10-175954),and pyrimidine (Published Japanese Translation of International Publication 7-500811), etc.
(8) nitrogen-containing condensed heterocyclic compounds such as indazole (JP-A Hei 6-287180), benzimidazole (JP-A Hei 6-305964), quinoline (JP-A Hei 3-161441), etc.
(9) sulfur- and nitrogen-containing condensed heterocyclic compounds such as benzothiazole (JP-A Hei 6-305964), etc.
(10) sulfur-containing condensed heterocyclic compound such as benzothiophene (JP-A Hei 7-196498), etc.
(11) oxygen-containing condensed heterocyclic compounds such as benzopyran (JP-A Hei 3-204874; JP-A Hei 4-308586), etc.
(12) nitrogenous compounds such as carbazoyl (JP-A Hei 2-156; JP-A Hei 2-753), carbazic acid (JP-A Hei 2-167264), hydrazine (JP-A Hei 3-148220), etc.
(13) quinones such as benzoquinone (JP-A Hei 9-315960), and hydroquinone (JP-A Hei 5-9114), etc.
(14) aliphatic dicarboxylic acids (JP-A Hei 1-56614; JP-A Hei 5-310565)
(15) silicone containing compounds (JP-A Sho 62-249709)
(16) organic germanium compounds (JP-A Hei 2-62885; JP-A Hei 5-255130; JP-A Hei 7-247296; JP-A Hei 8-59485)
(17) flavonoids (JP-A Hei 3-240725; JP-A Hei 7-206838; JP-A Hei 9-241165; WO 94/04520)
(18) alkylamines (JP-A Hei 6-206818; JP-A Hei 9-59233; JP-A Hei 9-40626; JP-A Hei 9-124471)
(19) amino acids (Published Japanese Translation of International Publication 4-502611; Published Japanese Translation of International Publication 7-503713)
(20) aromatic compounds such as ascochlorin (JP-A Hei 6-305959), benzoic acid (WO 91/11997), pyrrolo-naphthyridinium (JP-A Hei 10-158265), etc.
(21) polypeptides (Published Japanese Translation of International Publication 7-500580)
(22) vitamins such as pyridoxamine (WO 97/09981), etc.
(23) SH group-containing compounds such as glutathione, cysteine, N-acetylcysteine, etc.
(24) SH group-containing proteins such as reduced albumin, etc.
(25) tetracyclines (JP-A Hei 6-256280)
(26) chitosans (JP-A Hei 9-221427)
(27) tannins (JP-A Hei 9-40519)
(28) quaternary ammonium ion-containing compounds
(29) biguanides such as metformin, phenformin and buformin
(30) polymer compounds such as ion exchange resins
(31) inorganic compounds such as activated carbon, silica gel, alumina and calcium carbonate.

Most of the above compounds are generally known as Maillard reaction inhibitors. Maillard reaction means a non-enzymatic glycation reaction between a reducing sugar such as glucose, and an amino acid or protein. Focusing on a phenomenon of brown coloration in a mixture consisting of amino acid and reducing sugar upon heating, Maillard reported this reaction in 1912 (Maillard, L. C., Compt. Rend. Soc. Biol., 72: 599 (1912). Maillard reaction is involved in brown coloration of food, generation of aromatic components and taste, and protein denaturation during heating or storage. Therefore, this reaction has been mainly studied in the field of food chemistry.

In 1968, glycated hemoglobin (HbA1c), a micro fraction of hemoglobin, was identified *in vivo,* which was found to increase in patients with diabetes (Rahbar. S., Clin. Chim. Acta, 22: 296 (1968)). These findings helped launch a wave of interest in the significance of the *in vivo* Maillard reaction and the participation of the reaction in the onset of adult diseases, such as diabetic complications and arteriosclerosis as well as the progress of aging. Agents inhibiting the *in vivo* Maillard reaction were explored intensively, resulting in the discovery of the above-mentioned compounds as agents inhibiting the Maillard reaction.

However, it was not known that such Maillard reaction inhibitors are capable of improving the carbonyl-stress state in peritoneal-dialysis patients by eliminating carbonyl compounds from the blood.

There is no particular limitation on the type of carrier to be used for the immobilization of the carbonyl compound-trapping agent of the present invention, as long as it is insoluble in blood and harmless to the human body, and is also safe and stabile as a material directly contacting blood. Specifically, such carriers include, for example, synthetic or naturally occurring organic polymer compounds, inorganic materials such as glass beads, silica gel, alumina, and activated carbon, and those of which surfaces are coated with polysaccharide or synthetic polymer.

A carrier comprising a polymer compounds is exemplified by a polymethyl methacrylate, polyacrylonitrile, polysulfone, vinyl, polyolefin, fluorine, polyester, polyamide, polyimide, polyurethane, polyacryl, polystyrene, polyketone, silicon, cellulose, chitosan; specifically, polysaccharides such as agarose, cellulose, chitin, chitosan, sepharose, dextran, etc. and derivatives thereof, and polyester, polyvinyl chloride, polystyrene, polysufone, polyethersulfone, polypropylene, polyvinyl alcohol, polyarylether sulfone, polyacrylic ester, polymethacrylic ester, polycarbonate, acetylated cellulose, polyacrylonitrile, polyethylene terephthalate, polyamide, silicone resin, fluororesin, polyurethane, polyetherurethane, and polyacrylamide and derivatives thereof. These polymer materials can be used alone or in a combination of two or more types of polymers. In the latter case, the carbonyl compound-trapping agent is immobilized on at least one of the polymers. The immobilized carbonyl compound-trapping agent is used alone or in a combination of two or more types of compounds. Also, it is possible to add an appropriate modifier to these polymer materials or, subject them to denaturation treatment such as closs-linking by irradiation or peroxide.

There is no restriction on the shape of carrier. For example, the carrier can be membrane-like, fiber-like, granular-shaped, hollow fiber-like, non-woven fabric-like, porous, or honeycomb-shaped. The carrier's area of contact with the peritoneal dialysate can be controlled by varying the thickness, surface area, diameter, length, shape, and/or size of the carrier.

The carbonyl compound-trapping agent can be immobilized on the above-mentioned carrier by using known methods, such as physical adsorption, specific biochemical binding reaction, ion binding, covalent bonding, grafting, etc. If necessary, a spacer can be inserted between the carrier and the carbonyl compound-trapping agent. When the carbonyl compound-trapping agent is toxic, the amount released becomes a vital issue. Thus, it is preferred that the carbonyl compound-trapping agent is immobilized on the carrier by a covalent bond so as to minimize the amount released. Functional groups on the carrier are utilized for covalently bonding the carbonyl compound-trapping agent thereto. The functional group used is, for example, hydroxyl group, amino group, aldehyde group, carboxyl group, thiol group, silanol group, amide group, epoxy group, succinylimino group, etc.; however, the functional group is not limited to these groups. As examples of covalent bonds, ester bond, ether bond, amino bond, amid bond, sulfide bond, imino bond, disulfide bond, or the like can be given.

A commercially available product, for example polystyrene carrier having sulfonylhydrazine groups (PS-TsNHNH2, ARGONAUT TECHNOLOGIES CO.), can be used as a carrier for immobilizing carbonyl compound-trapping agent.

The carrier with the immobilized carbonyl compound-trapping agent of the present invention can be sterilized by an appropriate sterilization method selected from known sterilization methods depending upon the types of carbonyl compound-trapping agent and carrier used. The sterilization method includes, for example, autoclaving, gamma-ray irradiation, gas sterilization, etc.

Carbonyl compound-trapping agent-immobilized carriers could be contacted with blood in various ways. Examples are: the method where collected patient blood is infused into a blood bag filled with carbonyl compound-trapping agent-immobilized carriers, and trapping the carbonyl compounds in patient blood; the method where blood is circulated in a column filled with bead carriers or fiber carriers, or the like, on which a carbonyl compound-trapping agent has been immobilized, etc. Not only whole blood, but also seperated blood plasma may be used for the treatment. The treated blood may be returned to the patient or, if required, may be stored in a blood bag, or the like. It is also possible to trap carbonyl compounds that generate and accumulate in blood in blood bags during storage, by including carriers on which carbonyl compound-trapping agents are immobilized within the blood bags.

The contact between blood and carriers on which a carbonyl compound-trapping agent of this invention has been immobilized can be carried out during the blood purification step, including hemodialysis, blood filtration, blood filtration dialysis, blood adsorption, and blood plasma separation.

For example, both hemodialysis and trapping of carbonyl compounds can be carried out simultaneously in hemodialysis patients, by placing carriers on which a carbonyl compound-trapping agent has been immobilized in the hemodialysis circuit. In this case, it is preferable to use the.hemodialysis membrane as the carrier on which a carbonyl compound-trapping agent has been immobilized. Known types of dialysis membranes can be used as carriers. Examples are, cellulose derivatives such as regenerated cellulose, and cellulose triacetate; and polymethyl methacrylate, polyolefin, polysulfone, polyacrylonitrile (PAN), polyamide, polyimide, polyether nylon, silicon, and polyester copolymers, but are not limited thereto. As shown in the Examples, when polysulfone is used as the dialysis membrane, there was a decrease in the carbonyl intermediate product (pentosidine) level. Thus, among the above-mentioned dialysis membranes, it is particularly preferable to use a polysulfone membrane as the carrier. Instead of using a dialysis membrane as a carrier, a column filled with carriers on which a carbonyl compound-trapping agent has been immobilized may indeed be placed in the hemodialysis circuit as described above. Through contacting a patient's blood with carriers on which a carbonyl compound-trapping agent has been immobilized, carbonyl compounds are trapped from the blood, their damaging activity towards the living body is eliminated, and they become nontoxic. An anticoagulant may be used together to prevent blood-clotting in an extracorporeal circulation. Such anticoagulants include, for example, heparin, low-molecular-weight heparin, and Futhan (Nafamostat mesilate). These may be immobilized on carriers.

It is predicted that there maybe some cases where carbonyl compounds in patient blood are not completely treated during dialysis if the quantity of trapping agent used during the contact with blood is too small. Since pre-determination of the quantity of carbonyl compounds in the patient blood is particularly difficult, it is effective to maintain the activity of as many trapping agents as possible within a range that ensures the safety of the patient. The dose of a trapping agent can be adjusted by altering the quantity of trapping agent immobilized on the carriers, or the dose of carriers on which the trapping agent has been immobilized.

In addition to the organic compounds represented by the above Maillard reaction inhibitors, polymer compounds such as ion exchange resin, or inorganic compounds such as activated carbon, silica gel, alumina, and calcium carbonate can also be used as carbonyl compound-trapping agents of the present invention. These compounds, which are known as filling agents used for chromatography, can trap carbonyl compounds due to their adsorption capability. Such compounds themselves can function as carriers, and therefore, for example, a filtrator installed within an extracorporal blood circulation circuit can be filled with them for use. Such a compound can also be utilized as a "carbonyl compound-trapping agent" comprised in the an agent for improving carbonyl stress state of the present invention. In this case, such compounds themselves function as carriers on which a carbonyl compound-trapping agent has been immobilized as described above. Alternatively, another carbonyl compound-trapping agent can be further immobilized on carriers themselves having the capability of trapping carbonyl compounds.

An adsorbing-type blood-purification device is known, in which activated carbon is used. This adsorbing-type blood-purification device is used for a supplementary method associated with hemodialysis for the purpose of blood purification in drug poisoning and hepatic coma, and the removal of various intrinsic and extrinsic toxins and vasoactive substances increased in the earlier phases of onset of acute renal failure associated with multi-organ failure. However, it has been completely unknown that such an adsorbing-type blood-purification device is effective as a carbonyl compound-trapping agent.

### Brief Description of the Drawings

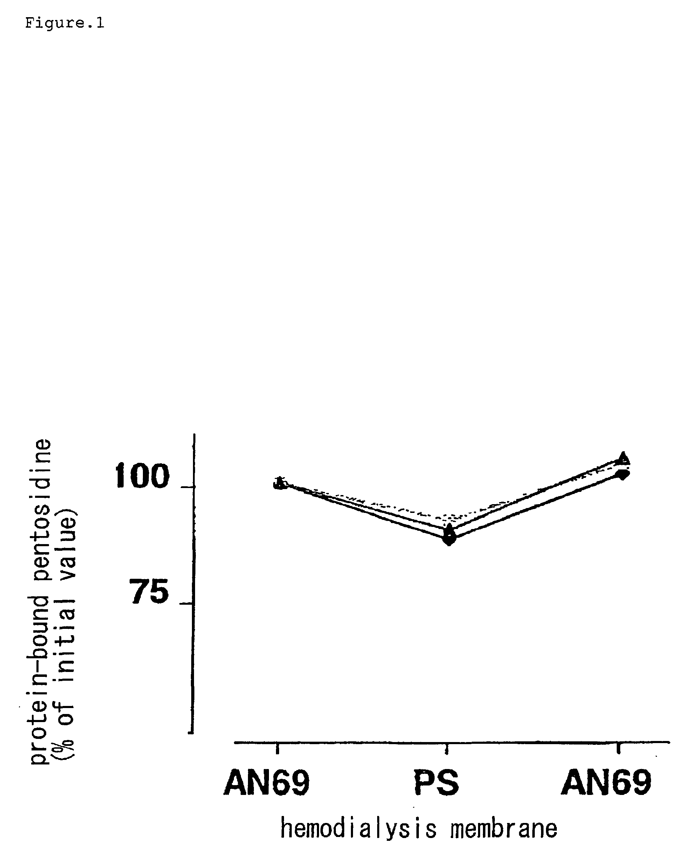

Figure 1 shows the effect of changing the type of hemodialysis membrane on blood plasma pentosidine levels (pmol/mg protein) in 3 patients. Each result is shown in a % value relative to the corresponding initial value (41.8 pmol/mg protein for patient 1 (◇), 22.1 pmol/mg protein for patient 2 (□) or 28.5 pmol/mg protein patient 3 (Δ)). Each value obtained is an average value of two samples collected 2 weeks after the end of each dialysis period (in the -2^{nd} and 0^{th} week of dialysis by AN69; in the 8^{th} and 10^{th} week after the switch to PS; in the 14^{th} and 16^{th} weeks after the returning to AN69).
Figure 2 shows the suppression effect on the pentosidine level of blood plasma in dialysis patients by the incubation with beads on which a carbonyl compound-trapping agent had been immobilized.
Figure 3 shows the carbonyl compound-trapping action by activated carbon in a dicarbonyl compound solution.
Figure 4 shows the dicarbonyl compound-trapping action by activated carbon in a peritoneal dialysate.
Figure 5 shows the activated carbon-mediated suppression effect on pentosidine generation when blood plasma from a dialysis patient was incubated at 37°C.
Figure 6 shows the removal of carbonyl compounds in blood plasma of a renal failure patient by activated carbon, or sulfonyl hydrazine-linked polystyrene beads. In this diagram, the ordinate shows the carbonyl compound concentration.
Figure 7 shows the removal of carbonyl compounds in blood plasma of a renal failure patient by aminoguanidine. In this diagram, the ordinate shows the carbonyl compound concentration.
Figure 8 shows a method for preparing diaminoguanidine-linked polyamide.
Figure 9 shows the removal of carbonyl compounds in a dicarbonyl compound solution by diaminoguanidine-linked polyamide.

### Best Mode for Carrying out the Invention

The present invention is specifically illustrated below with reference to Examples, but it is not to be construed as being limited thereto.

### [Example 1] The influence of the type of hemodialysis membrane on blood plasma pentosidine level

### 1. Patients

Belgian patients (n=29) and Japanese patients (n=97), 126 patients (69 males and 57 females) in total, who had been subjected to hemodialysis 3 times a week, were tested. They were 61.2±13 (standard deviation) years old on average. Only two of them were affected with mild type-II diabetes mellitus. Each patient used the same type of hemodialysis membrane for at least 3 months (or, 2 or 3 patients used the same type of membrane for less than 3 months, but ever since initiating hemodialysis). In 26 of 29 Belgian patients, dialysis membranes were reused, but there was no reuse in Japanese patients. Data of residual diuresis (ml/day), surface area of dialysis membrane, and duration of hemodialysis were obtained from clinical records of each patient.

### 2. Types of membranes

The following hemodialysis membranes were used: high-flux (UF - index>10 ml/mmHg/h) AN69 (Hospal; France) (group of AN69); high-flux polysulfone (Fresenius; Germany) (group of PS); high-flux polysulfone (Asahi Medical; Japan) (group of APS); high-flux polymethylmethacrylate (Toray; Japan) (group of PMMA); and low-flux cellulosic (Asahi Medical; Japan) (group of cellulose).

### 3. Blood plasma samples

Blood plasma samples were collected from all the 126 patients prior to the first hemodialysis, and then from 66 patients weekly after dialysis.

All the samples were immediately subjected to centrifugal separation. The blood plasma samples frozen at -20°C were tested as follows:

### 4. Quantification of total pentosidine and free pentosidine

For quantification of total pentosidine, a sample (50 µl) was lyophilized, dissolved in 100 µl of 6N HCl, encapsulated with nitrogen gas and then incubated at 110°C for 16 hours, neutralized with 100 µl of 5N NaOH and 200 µl of 0.5 M phosphate buffer (pH 7.4), subsequently filtered through a filter with a pore size of 0.5 µm, and diluted 20 times with PBS. For quantification of the free pentosidine, the sample (50 µl) was mixed with an equal amount of 10% TCA, and then centrifuged at 5000 × g for 10 minutes. The supernatant was filtered through a filter with a pore size of 0.5 µm and diluted 4 times with distilled water.

Pentosidine in each of these samples was analyzed by reverse phase HPLC using a C18 reverse phase column (Waters, Tokyo, Japan) (Miyata, T. et al., J. Am. Soc. Nephrol., 7: 1198-1206, 1996). The effluent was monitored with a fluorescence detector (RF-10A; Shimadzu) at an excitation/detection wavelength =335/385nm. A standard curve was prepared by using synthetic pentosidine.

The level of protein-bound pentosidine (pentosidine/protein) (pmol/mg protein) was calculated by [total pentosidine in blood plasma (pmol/ml) - free pentosidine (pmol/ml)]/[blood plasma protein concentration (mg/ml)].

### 5. Statistical analysis between groups of patients

The respective values, including quantification of the pentosidine level, are indicated as mean ± standard deviation or percentage (%). The data of residual diuresis was log-transformed. Through one-way analysis of variance (one-way ANOVA) (with F-test), individual data and the pentosidine level were compared between groups of hemodialysis patients using different dialysis membranes. Furthermore, the groups of dialysis membranes were compared and analyzed by using Bonferroni t-test. Through chi 2 test, the degree of residual diuresis was compared between the various groups.

The result of cross-sectional analysis among the five groups of patients is shown in Table 1. There were no significant age-related differences among the groups. The blood plasma protein level was higher in the AN69 and cellulosic groups. In APS group, the area of the dialysis membrane was larger, and both the period of hemodialysis prior to the study and duration of a single dialysis were longer. On the other hand, residual diuresis was higher in the PS group.

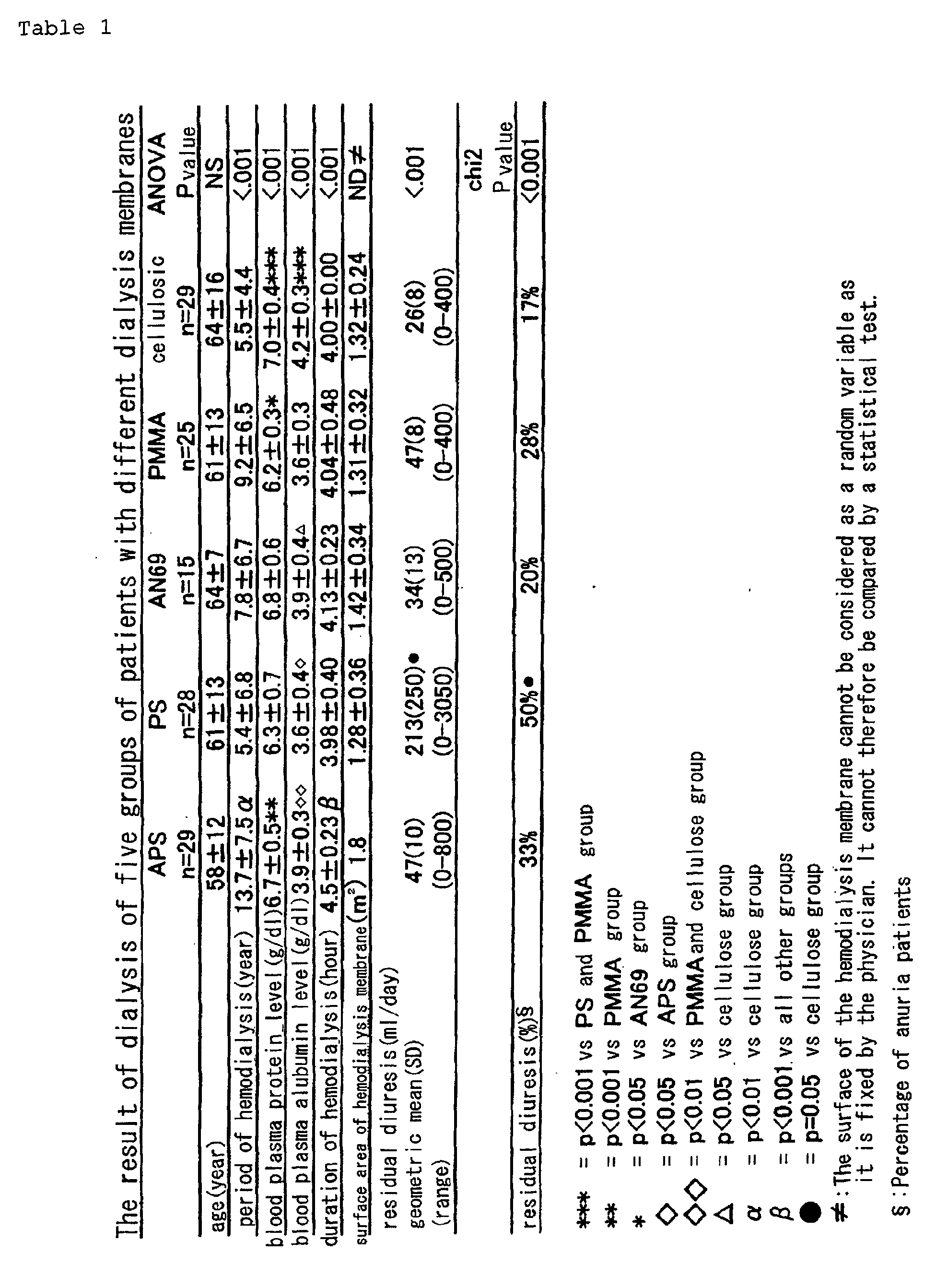

Before dialysis, the levels of protein-bound pentosidine and of free pentosidine in blood plasma were similar among the AN69, PMMA, and cellulosic groups, but were significantly lower in the PS and APS groups. There was no significant difference between the PS and APS groups (Table 2).

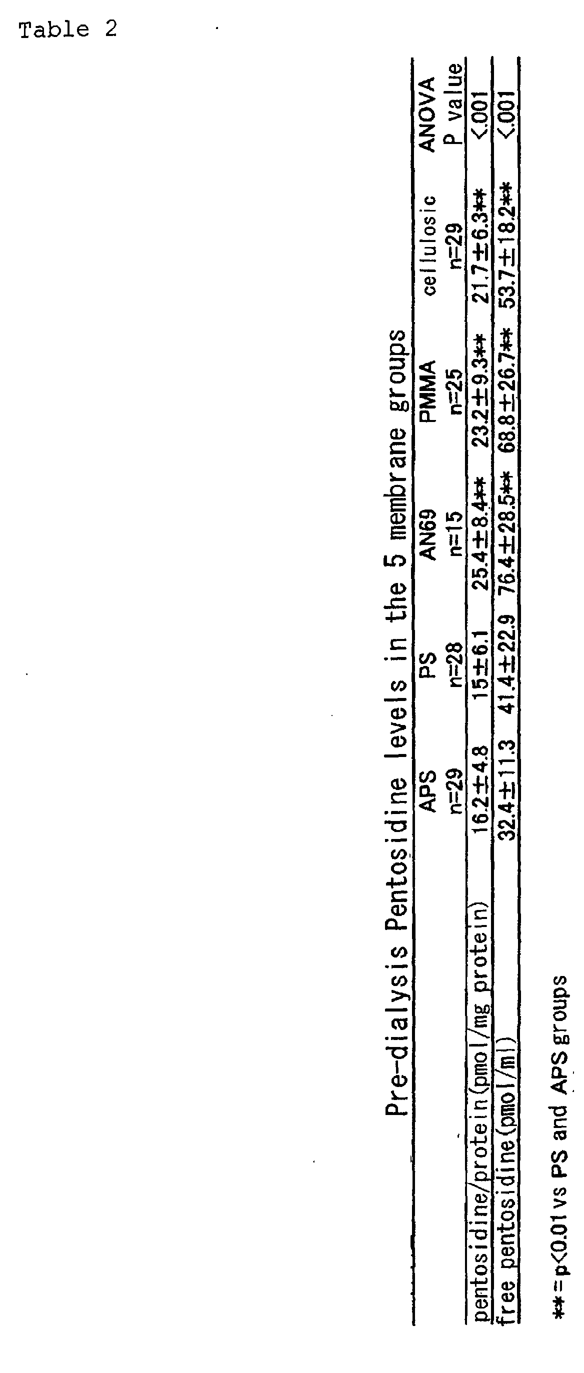

Univariate analysis of a variety of factors that may influence predialysis blood plasma pentosidine, indicated that residual diuresis significantly affected protein-bound and free pentosidine: the higher the residual diuresis, the lower the pentosidine level. No correlation was found between pentosidine level and blood plasma protein level, albumin level, age, or dialysis history (Table 3). Relating to polysulfone (PS and APS groups) groups, the predialysis pentosidine level in Belgian patients or Japanese patients subjected to dialysis with polysulfone membranes from Fresenius or Asahi Medical co. were similar to each other (Table 4).

**Table 3**

| Univariate analysis of the relationship between pentosidine level and potentially explanatory continuous variables: r values | | | | | |
|---|---|---|---|---|---|
| | log.diuresis | Total protein | Alubumin | Age | Duration |
| pentosidine/protein | -0.28 | -0.08 | 0 | 0.14 | 0.03 |
| free pentosidine | -0.36 | 0.01 | -0.12 | 0.15 | 0.02 |
| ** = p<0.01 *** = p<0.001 | | | | | |

**Table 4**

| Pentosidine levels and residual diuresis in polysulfone groups, according to polysulfone brand and/or country of patients | | | | |
|---|---|---|---|---|
| | Fresenius Belgium | Fresenius Japan | Asahi Japan | P value |
| pentosidine/protein (pmol/mg protein) | 14.6±6.2 | 15.3±6.3 | 16.2±4.8 | NS |
| free pentosidine(pmol/ml) | 37.3±19.6 | 45.4±25.9 | 32.4±11.3 | NS |
| residual diuresis (ml/day) | 938(23) | 49(14) | 47(10) | 0.004 |

As described above, it was revealed that pentosidine levels were lower in dialysis patients using polysulfone dialysis membranes than those in dialysis patients using other dialysis membranes. Decreases in pentosidine levels were seen in patients dialyzed with a polysulfone membrane, independent of the patients' country or the manufacturer of the dialysis membranes. Furthermore, although patients subjected to dialysis with polysulfone dialysis membrane from Ashahi Medical were substantially anuric, pentosidine levels were similarly lower. In the group using polysulfone from Fresenius, there was no alteration in statistical significance in the difference of pentosidine level, even when patients with urinary volumes greater than 300 ml/min were excluded.

Because AN69 is also high-flux type and pentosidine levels before dialysis in dialysis with high-flux AN69 and low-flux cellulosic showed similar results, the difference in pentosidine level is estimated to be independent of the removing capacity of dialysis membrane.

The relationship between protein-corrected pentosidine level or free pentosidine level and residual diuresis was analyzed by linear regression analysis. The effect of each explanatory variable on dependent variable (protein-corrected pentosidine level and free pentosidine level) was tested by variable selection - multiple regression analysis (Forward stepwise multiple regression analysis). All the analyses were carried out by using BMDP statistics software (BMDP is the trade mark of New System Professional Edition: Statistical Solutions Inc., University of California Press, Berkeley, 1995). A P value less than 0.05 was considered significant.

The analysis result showed that dialysis membrane type and residual diuresis were the two sole independent determinants of protein-bound and free pentosidine levels (Table 5). Considering the fact that none of the interactions were significant, the influence of residual diuresis on pentosidine level was not affected by dialysis membrane type.

**Table 5**

| The variable selection-multiple regression analysis of determinants of pentosidine levels (Forward stepwise multiple regression analysis) | | | | |
|---|---|---|---|---|
| | pentosidine/protein | | free pentosidine | |
| | Increase in R | P value | Increase in R | P value |
| membrane type | 0.53 | <0.001 | 0.59 | <0.001 |
| log. diuresis | -0.21 | <0.001 | -0.36 | <0.001 |
| total protein | -0.17 | NS | 0.05 | NS |
| albumin | -0.05 | NS | -0.1 | NS |
| age | 0.12 | NS | 0.16 | NS |
| period of hemodialysis | -0.01 | NS | -0.08 | NS |

### 6. The effect of dialysis membrane on pentosidine levels before and after hemodialysis

To further analyse the mechanism underlying dialysis membrane effect on predialysis pentosidine levels, pentosidine levels before and after dialysis were determined for four groups of patients with high-flux polysulfone (Fresenius), AN69, PMMA, or low-flux cellulosic dialysis membranes (Table 6).

**Table 6**

| Influence of a single hemodialysis session on pentosidine level | | | | |
|---|---|---|---|---|
| | PS n=14 | AN69 n=15 | PMMA n=9 | cellulosic n=28 |
| pentosidine/protein(pmol/mg protein) | | | | |
| Pre | 14.6±6.2 | 25.4±8.4 | 24.3±8.5 | 21.8±6.4 |
| Post | 13.8±6.6 | 23.4±5.6 | 22.8±8.3 | 22.1 ±5.8 |
| free pentosidine(pmol/ml) | | | | |
| Pre | 37.3±19.6 | 76.4±28.5 | 70.3±26 | 53.5±18.5 |
| Post | 10.9±6.6 | 17.5±4.9 | 21.7±7 | 14.7±6.9 |
| (reduction ratio) (%) | (71±11) | (76±7) | (67±9) | (73±6) |

As predicted by the above experiment, the level of protein-bound pentosidine was almost unaltered and also was independent of the dialysis membrane type. Although only the level of free pentosidine markedly decreased, the percentage, which was between 76%(AN69) and 67%(PMMA) was similar in all the groups. There was no significant difference among the groups. Thus, it was revealed that the lower predialysis pentosidine levels observed in dialysis patients using polysulfone membranes could not be accounted for by differences in the dialysis capacity of the membranes.

The present inventors previously showed that hemodialysis itself did not affect the total pentosidine or protein-bound pentosidine levels (Miyata, T. et al., Kidney Int., 51: 880-887, 1997). This finding agrees with the fact that 95% of pentosidine bind with albumin that cannot be eliminated through dialysis (Miyata, T. et al., J. Am. Soc. Nephrol., 7: 1198-1206, 1996). This finding is supported by the result obtained in the above Example with the four different types of dialysis membranes. On the contrary, the amount of free pentosidine decreased by hemodialysis, and a similar phenomenon was observed with every dialysis membrane. This result is predictable by the molecular weight of free pentosidine (379 Da). The fact that pentosidine levels before and after hemodialysis were similar for all the dialysis membranes indicates that not only passive transport, but also absorption of pentosidine occurs during dialysis with polysulfone or other dialysis membranes. The level of absorption of radio-labeled free pentosidine with cellulosic membrane or polysulfone membrane *in vitro* is very small, and in reality, there was no difference.

Thus, the decreased predialysis pentosidine level cannot be explained by the fact that the polysulfone membrane enhances removal of pentosidine . There may be another possibility that the dialysis with polysulfone membrane relates to the suppression of pentosidine production.

As pointed out previously, pentosidine level reflects the concentration of carbonyl intermediates derived from carbohydrates. The polysulfone membrane has the specific effect of removing these carbonyl compounds, and thus, it may be having an effect on pentosidine production. Alternatively, there may be also the possibility that the polysulfone membrane reduces oxidative stress assumed to be associated with uraemia (Miyata, T. et al., Kidney Int., 54; 1290-1295, 1998; Miyata, T. et al., Kidney Int., 51: 1170-1181, 1997; Loughrey, CM. et al., Q. J. Med., 87: 679-683, 1994; Ueda, Y. et al., Biochem. Biophys. Res. Commun., 245: 785-790, 1998; Kumano, K. et al., Adv. Perit. Dial., 1992; 8: 127-130; Witko-Sarsat, V. et al., Kidney Int., 49: 1304-1313, 1996). There is also the possibility that the decrease in oxidative stress suppreses the production of carbonyl compounds, and as a result, the generation of pentosidine is decreased (Miyata, T. et al., Kidney Int., 51: 1170-1181, 1997).

### 7. The effect of switching the dialysis membrane on the pentosidine level

To verify the specific pentosidine-lowering effect of polysulfone, a longitudinal analysis was conducted in three anuria patients subjected to long-term (>5 years) AN69 dialysis. The patients were switched to a polysulfone (Fresenius) dialysis membrane of similar surface area for 10 weeks, and subsequently, the membrane was returned to AN69. Predialysis samples were collected every 2 weeks before the switch to PS (2 samples), during PS dialysis (5 samples), and 14-16 weeks after the return to AN69 (2 samples).

It was found that protein-bound pentosidine level of each patient gradually decreased after switching to PS dialysis, and subsequently, due to the resumption of AN69 dialysis, the level returned to that when using AN69 prior to the switch to PS (Figure 1).

The decrease in pentosidine level observed in the longitudinal study in patients, which was carried out by switching AN69 to PS dialysis, is only one third of the difference (10.4 compared to 3.6 pmol/mg protein) between the PS and AN69 groups observed in the cross-sectional study. This discrepancy may be due to the fact that the observation after the transfer to PS dialysis lasted only 10 weeks. If polysulfone reduces the rate of pentosidine generation, it will be possible to explain the fact that protein-bound pentosidine decreased so slowly. Under such circumstances, the decrease in protein-bound pentosidine level may result from only protein metabolism. A similar observation was made after successful kidney transplantations. In such cases, the decrease in protein-bound pentosidine was considerably slower than the decrease in blood plasma β2-microglobulin, suggesting that the decrease occurs very slowly, and the decomposition of protein-bound pentosidine is very slow(Miyata, T. et al., Kidney Int., 51: 880-887, 1997; Hricik, DE. et al., Clin. Transplantation, 10: 568-573, 1996). [Example 2] The removal of blood carbonyl compounds by carriers on which a carbonyl compound-trapping agent has been immobilized

A cross-linked polystyrene resin bound to a sulfonyl hydrazine group (PS-TsNHNH2, ARGONAUT TECHNOLOGIES) was used as a carbonyl compound-trapping bead to study its effect in removing blood carbonyl compounds. Blood plasma from a dialysis patient and those supplemented with carbonyl compound-trapping beads were incubated at 37°C to test the pentosidine generation-suppressing effect. 100 µl of dimethylsulfoxide was added to the tube containing carbonyl compound-trapping beads to swell the beads, and then filter-sterilized blood plasma from a predialysis dialysis patient was added thereto. The mixture was incubated at 37°C for one week. After incubation, the beads were removed with a centrifugal filter having a pore size of 0.22 µm(Millipore, UFC30GV00). Then, 50 µl of 10% trichloroacetic acid was added to 50 µl of the bead-free solution, and the mixture was centrifuged to precipitate proteins. The resulting protein pellet was washed with 300 µl of 5% trichloroacetic acid and then dried. Subsequently, 100 µl of 6N Hcl was added to the pellet and heated at 110°C for 16 hours, and then, pentosidine was quantified by HPLC (Miyata, T. et al., 1996, J. Am. Soc. Nephrol., 7: 1198-1206, Miyata, T. et al., 1996, Proc. Natl. Acad. Sci. USA, 93: 2353-2358).

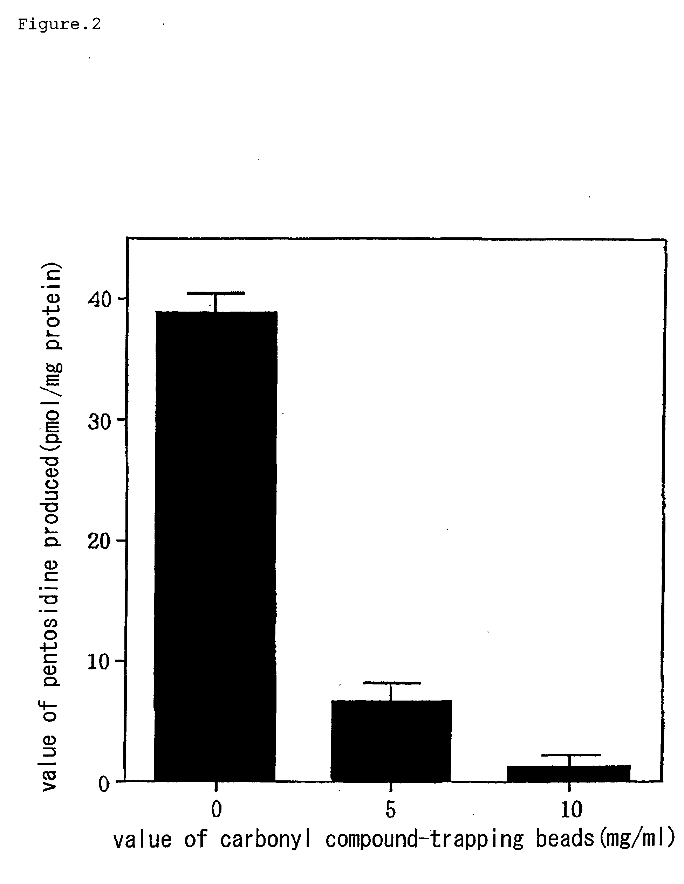

The amount of pentosidine generated during incubation at 37°C is shown in Figure 2. It was found that the addition of carbonyl compound-trapping beads suppressed the generation of pentosidine. In addition, the suppression of pentosidine generation depended on the amount of carbonyl compound-trapping beads added.

These results showed that blood carbonyl compounds could be removed by carriers on which the carbonyl compound-trapping agent had been immobilized. Further, it was revealed that polysulfone membrane was a particularly suitable hemodialysis membrane for improving carbonyl stress state.

### [Example 3] The removal of carbonyl compounds from a dicarbonyl compound solution by activated carbon

900 µl of a dicarbonyl solution, in which each of glyoxal, methylglyoxal, and 3-deoxyglucosone had been dissolved (100 µM each) in PBS (-), was added to a tube containing 25 mg or 50 mg of activated carbon (Wako Pure Chemical Industries) and the mixture was stirred with a rotator at room temperature for 19 hours. Then, the solution was filtered through a centrifugal filtration tube with a pore size of 0.22 µm (Millipore; UFC30GV00) and the concentrations of glyoxal, methylglyoxal, and 3-deoxyglucosone in the filtrate were measured by high-performance liquid chromatography.

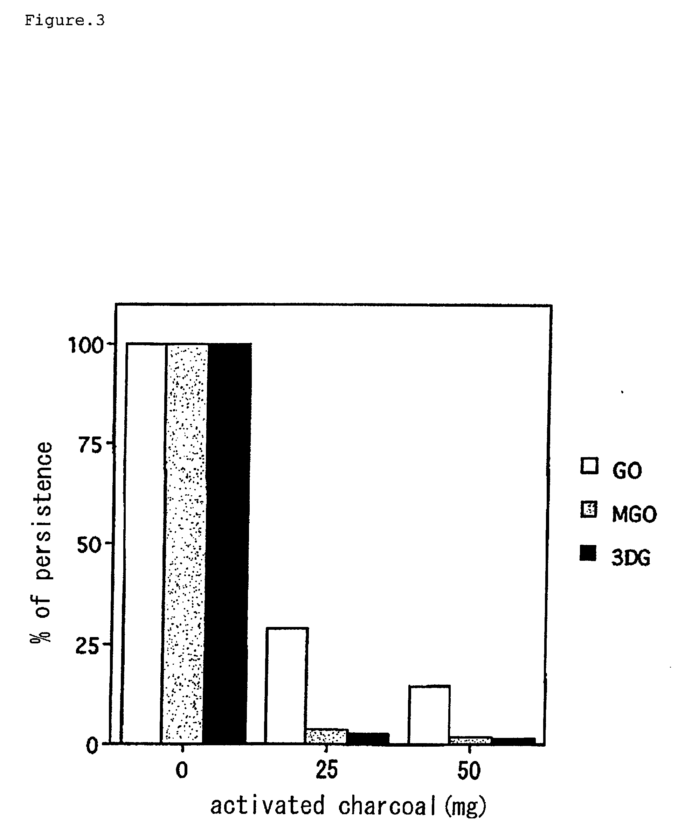

When 900 µl of the dicarbonyl solution was added to 25 mg of activated carbon, 71% of glyoxal, 96% of methylglyoxal, and 97% of 3-deoxyglucosone were trapped. When 50 mg of activated carbon was used, 85% of glyoxal, 98% of methylglyoxal, and 98% of 3-deoxyglucosone were trapped (Figure 3).

### [Example 4] The removal of dicarbonyl compounds from a peritoneal dialysate by activated carbon

Since typically, a peritoneal dialysate contains a high concentration of glucose, glucose-derived carbonyl compounds are produced during sterilization or storage. These carbonyl compounds are transferred into the living body during peritoneal dialysis, which is one factor causing carbonyl stress state. Thus, the effect of the carbonyl compound-trapping agent of the present invention in removing carbonyl compounds from a peritoneal dialysis liquid was evaluated.

900 µl of a peritoneal dialysate (Baxter Ltd.; Dianeal PD-4, 1.5) was added to a tube containing 25 mg or 50 mg of activated carbon and the mixture was stirred with a rotator at room temperature for 19 hours. Then, the solution was filtered through a centrifugal filtration tube with a pore size of 0.22 µm (Millipore; UFC30GV00), and the concentrations of glyoxal, methylglyoxal, and 3-deoxyglucosone in the filtrate were measured by high-performance liquid chromatography.

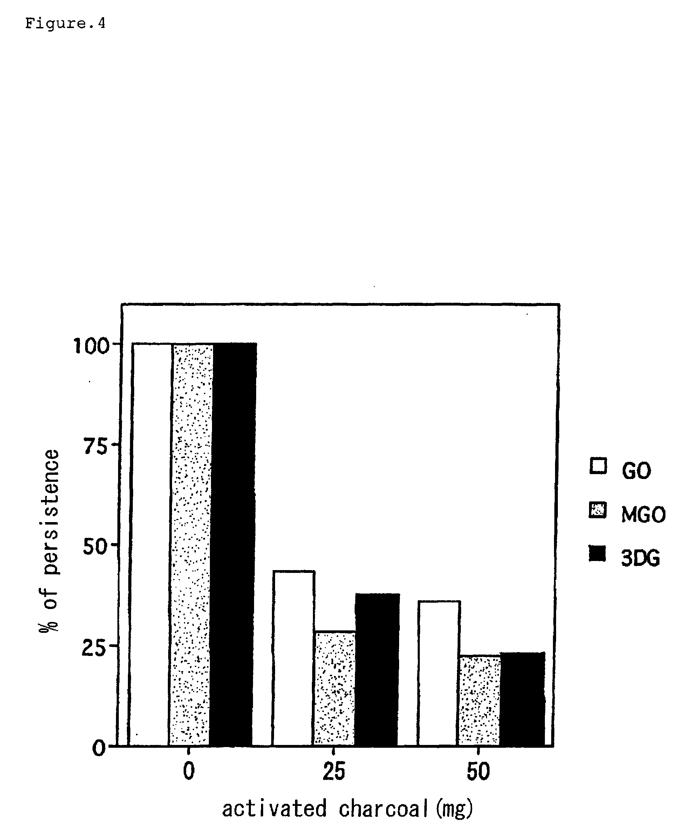

When 900 µl of peritoneal dialysate was added to 25 mg of activated carbon, 56% of glyoxal, 71% of methylglyoxal, and 62% of 3-deoxyglucosone was trapped. When 900 µl of peritoneal dialysate was added to 50 mg of activated carbon, 64% of glyoxal, 78% of methylglyoxal, and 77% of 3-deoxyglucosone were trapped (Figure 4).

### [Example 5] The pentosidine generation-suppressing effect of activated carbon when blood plasma from a dialysis patient was incubated at 37°C

250 µl of filter-sterilized blood plasma from a predialysis dialysis patient was added to a tube containing 12 mg of activated carbon suspended in PBS(-), and the mixture was incubated at 37°C for one week. After incubation, 50 µl of 12N HCl was added to 50 µl of supernatant obtained by centrifugation, and the mixture was heated at 110°C for 16 hours for hydrolysis. Then, pentosidine was quantified by high-performance liquid chromatography (Miyata, T. et al., 1996, J. Am. Soc. Nephrol., 7:1198-1206, Miyata, T. et al., 1996, Proc. Natl. Acad. Sci. USA., 93:2353-2358).

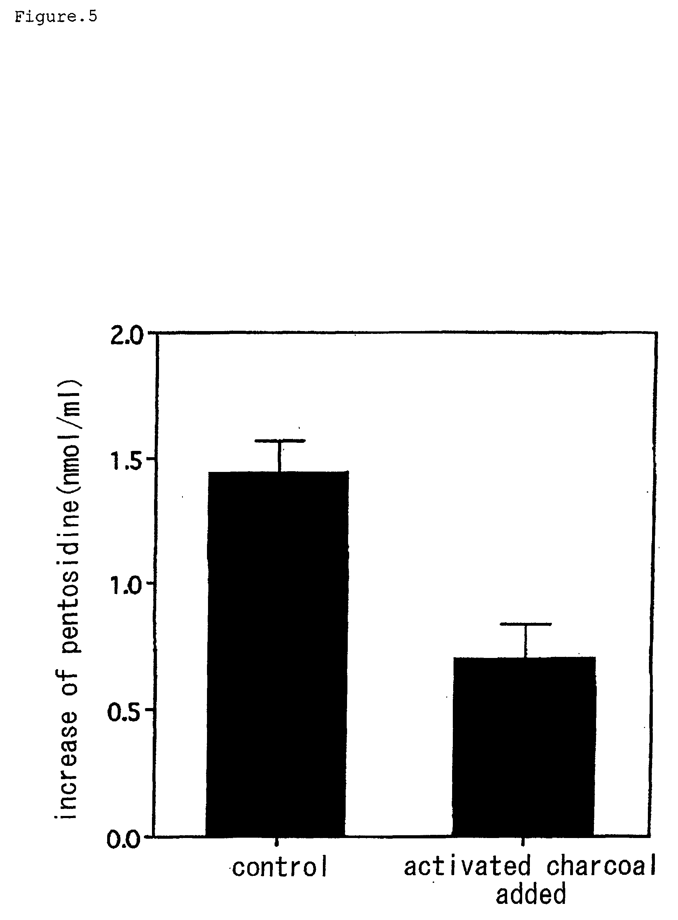

The amount of pentosidine generated by the incubation at 37 °C is shown in Figure 5. When compared with the control, 51% of pentosidine generation was suppressed by the addition of activated carbon. This suggested that carbonyl compounds that are precursors of pentosidine were adsorbed by activated carbon.

### [Example 6] The removal of carbonyl compounds from blood plasma by activated carbon

500 µl of blood plasma from a renal failure patient was added to a tube containing 20 mg or 50 mg of activated carbon (Wako Pure Chemical Industries), and then the mixture was stirred with a rotator at room temperature for 12 hours. After the separation of activated carbon by centrifugation, the concentrations of glyoxal and methylglyoxal in blood plasma were measured by high-performance liquid chromatography.

The concentrations of glyoxal and methylglyoxal in blood plasma were measured as follows. First, 300 µl of 0.67M perchloric acid was added to 200 µl of blood plasma, and then, the mixture was stirred and centrifuged to separate the supernatant. 20 µl of 1% o-phenylenediamine and 50 µl of 10 µM 2,3-butanedione (internal standard) were added to 150 µl of the supernatant. The mixture was stirred and incubated at 25°C for 1 hour. According to the method described by Ohmori et al. (Ohmori, S. et al., J. Chromatogr., 414: 149-155, 1987), quinoxaline derivatives generated via the reaction between glyoxal or methylglyoxal and o-phenylenediamine were separated for the quantification by HPLC with a reversed-phase column.

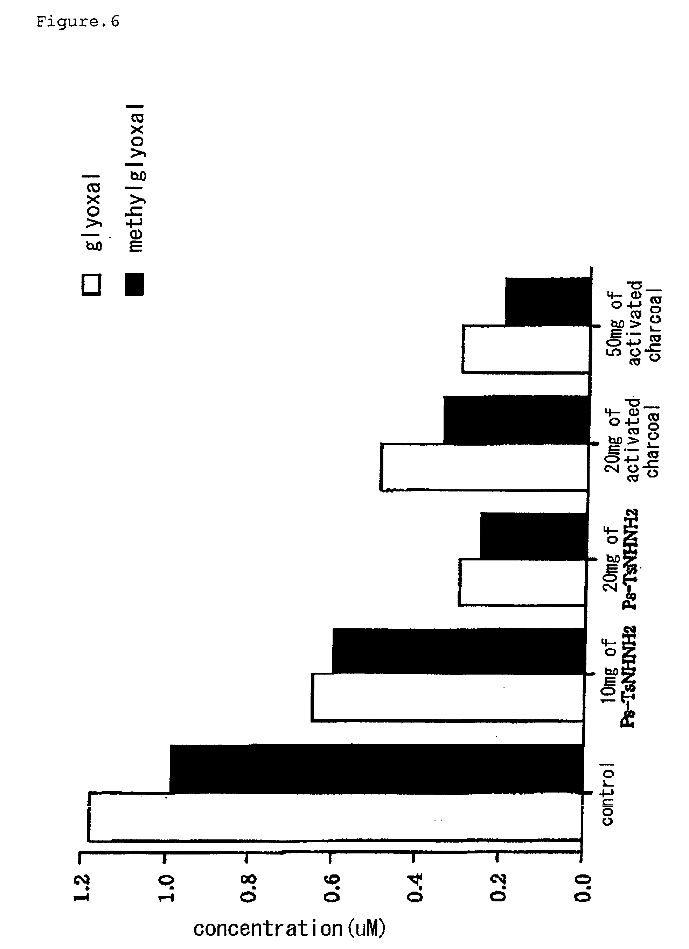

The result is shown in Figure 6. When 20 mg of activated carbon was added to blood plasma, 58% of glyoxal and 65% of methylglyoxal were trapped. When 50 mg of activated carbon was added, 75% of glyoxal and 80% of methylglyoxal was trapped.

### [Example 7] The removal of carbonyl compounds from blood plasma by sulfonyl hydrazine-linked polystyrene beads (Ps-TsNHNH₂)

500 µl of blood plasma from a renal failure patient was added to a tube containing 10 mg or 20 mg of sulfonyl hydrazine-linked polystyrene beads and then the mixture was stirred with a rotator at room temperature for 12 hours. After the separation of sulfonyl hydrazine-linked polystyrene beads by centrifugation, the concentrations of glyoxal and methylglyoxal in blood plasma were measured by high-performance liquid chromatography according to the same method as in Example 6. The result is shown in Figure 6. When 10 mg of sulfonyl hydrazine-linked polystyrene beads were added to blood plasma, 45% of glyoxal and 39% of methylglyoxal were trapped. When 20 mg of sulfonyl hydrazine-linked polystyrene beads were added, 75% of both glyoxal and methylglyoxal were trapped.

### [Example 8] The removal of carbonyl compounds from blood plasma by aminoguanidine

50 µl of a solution in which aminoguanidine (50 mM, or 100 mM) was dissolved in 0.1M sodium phosphate buffer (pH 7.4) was mixed with 450 µl of blood plasma from a renal failure patient, and the resulting mixture was left at room temperature for 12 hours. After 12 hours, the concentrations of glyoxal and methylglyoxal in blood plasma were measured by high-performance liquid chromatography according to the same method as in Example 6.

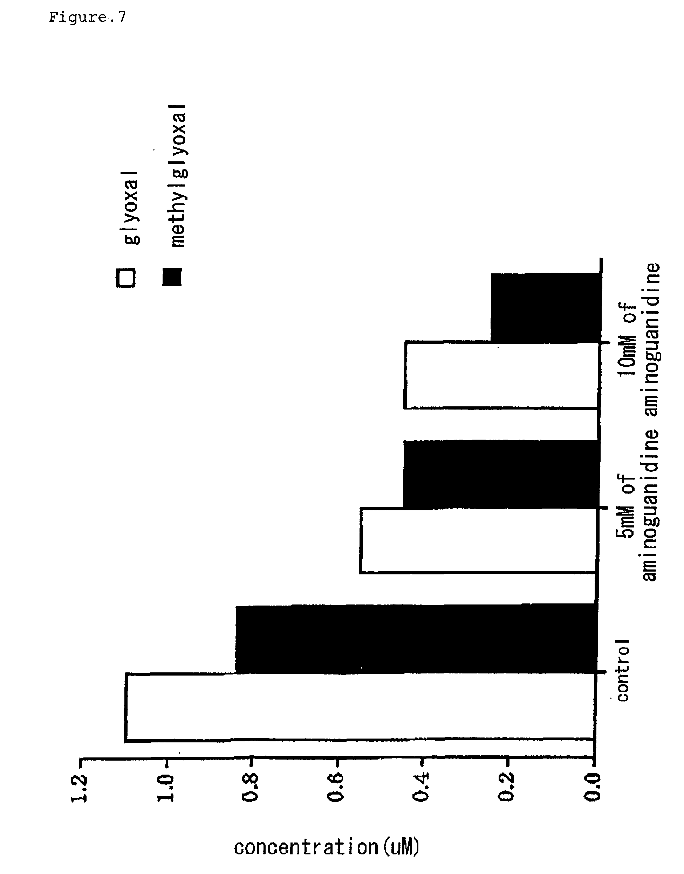

The result is shown in Figure 7. When aminoguanidine concentration in blood plasma was 5 mM, 50% of glyoxal and 46% of methylglyoxal were trapped. When aminoguanidine concentration was 10 mM, 58% of glyoxal and 70% of methylglyoxal were trapped.

### [Example 9] The removal of carbonyl compounds by carriers on which a carbonyl compound-trapping agent has been immobilized

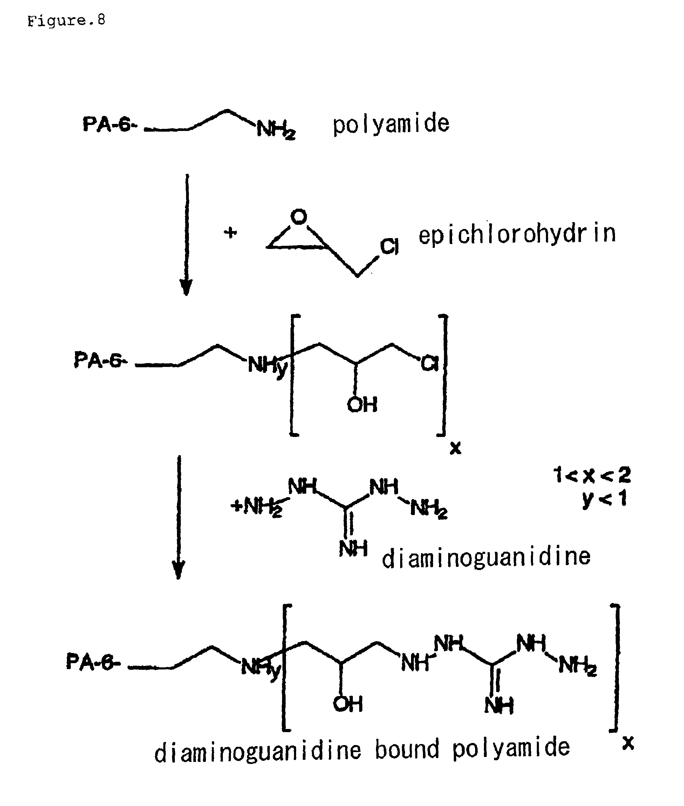

The action of removing carbonyl compounds by carriers, on which a carbonyl compound-trapping agent has been immobilized, was evaluated by using diaminoguanidine-linked polyamide. Diaminoguanidine-linked polyamide was prepared by reacting polyamide with epichlorohydrin and adding an aqueous solution of diaminoguanidine (pH 12) thereto, followed by incubation at 80°C for about 1 hour (Figure 8). After the reaction was completed, the resulting diaminoguanidine-linked polyamide was washed with water and then dried for use in the subsequent experiments.

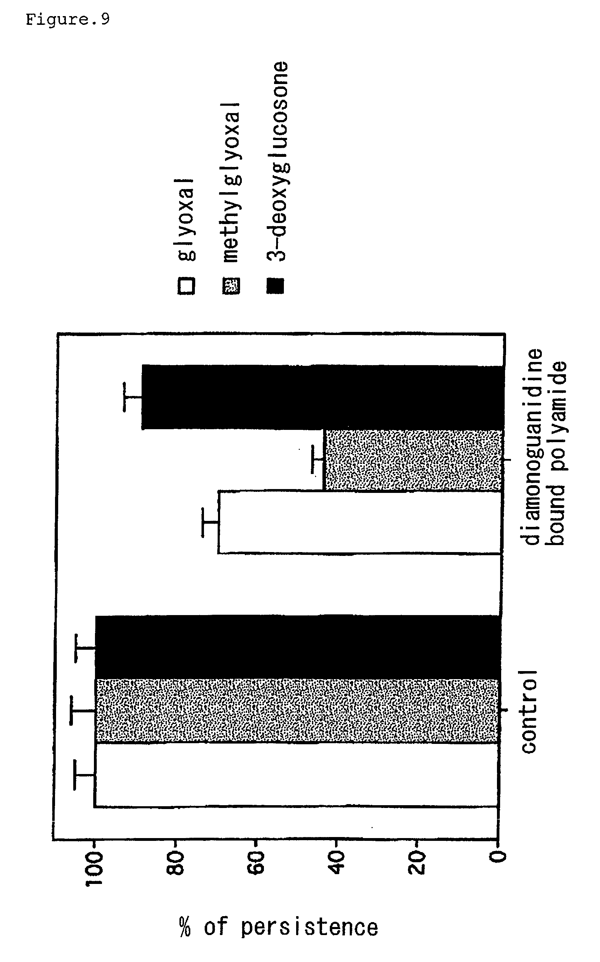

1 ml of a dicarbonyl compound solution (glyoxal, methylglyoxal, 3-deoxyglucosone; 1 µM each in PBS (pH 7.4)) was added to a tube containing 30 mg of diaminoguanidine-linked polyamide. The mixture was stirred with a rotator at room temperature (25°C) for 5 hours, and 100 µl of those were centrifuged. The residual glyoxal, methylglyoxal, and 3-deoxyglucosone in the supernatant were converted to derivatives thereof, and were determined by high-performance liquid chromatography. The result is shown in Figure 9. 30% of glyoxal, 56% of methylglyoxal, and 11% of 3-deoxyglucosone were trapped. When diaminoguanidine-free polyamide was used as a negative control under the same conditions, the above carbonyl compound-trapping action was not detectable.

The results described above verified that the carbonyl compounds could be removed effectively from the liquid by carriers on which the carbonyl compound-trapping agent has been immobilized.

### Industrial Applicability

The present invention enables effective removal of blood carbonyl compounds. The agent of the present invention for improving carbonyl stress state can be used readily by immobilizing it on a dialysis membrane for hemodialysis, or alternatively immobilizing it on other carriers and placing it within the blood circuit. Thus, the present invention makes it possible to ease damages caused by carbonyl compounds (i.e. carbonyl stress) from which renal failure patients have long suffered.

## Claims

1. A carbonyl compound-trapping agent that removes a blood carbonyl compound.

2. The carbonyl compound-trapping agent according to claim 1, wherein said agent is used in hemodialysis.

3. The carbonyl compound-trapping agent according to claim 1, wherein said agent is immobilized on a blood-insoluble carrier.

4. The carbonyl compound-trapping agent according to claim 3, wherein said carrier is a dialysis membrane.

5. The carbonyl compound-trapping agent according to claim 4, wherein said dialysis membrane is a polysulfone membrane.

6. The carbonyl compound-trapping agent according to claim 1, wherein said carbonyl compound-trapping agent is a Maillard reaction inhibitor.

7. The carbonyl compound-trapping agent according to claim 6, wherein said Maillard reaction inhibitor comprises at least one compound selected from the group consisting of aminoguanidine, pyridoxamine, hydrazine, SH group-containing compound, and derivatives thereof.

8. The carbonyl compound-trapping agent according to claim 1, wherein said agent comprises a compound that is insoluble in blood.

9. The carbonyl compound-trapping agent according to claim 8, wherein said compound that is insoluble in blood comprises at least one compound selected from the group consisting of an ion exchange resin, activated carbon, silica gel, alumina, and calcium carbonate.

10. An agent for improving the carbonyl stress state in a living body, wherein said agent comprises a carbonyl compound-trapping agent as an active ingredient.

11. An agent for improving the carbonyl stress state in blood, wherein said agent comprises a carbonyl compound-trapping agent as an active ingredient.

12. The agent for improving the carbonyl stress state according to claim 11, wherein said agent is immobilized within the blood circuit.

13. The agent for improving the carbonyl stress state according to claim 11, wherein the carbonyl compound-trapping agent is a Maillard reaction inhibitor.

14. The agent for improving the carbonyl stress state according to claim 13, wherein said Maillard reaction inhibitor comprises at least one compound selected from the group consisting of aminoguanidine, pyridoxamine, hydrazine, SH group-containing compound, and derivatives thereof.

15. A method for improving carbonyl stress state, wherein said method comprises the step of contacting, within the blood circuit, a patient's blood with a carbonyl compound-trapping agent.

16. The method according to claim 15, wherein said method comprises the step of immobilizing said carbonyl compound-trapping agent on a blood-insoluble carrier.
